# EUROPEAN PATENT APPLICATION

(11) **EP 4 613 729 A1**
(43) Date of publication of application: **10.09.2025**
(21) Application number: 23885628.0
(22) Date of filing: 25.10.2023
(51) Int. Cl.: C07C 29/153, B01J 23/46, B01J 23/50, B01J 23/75, B01J 23/80, B01J 23/83, B01J 23/89, B01J 23/656, B01J 23/755, B01J 23/882, B01J 23/888, B01J 37/02, B01J 37/04, B01J 37/16, C07B 61/00, C07C 29/36, C07C 31/08, C07C 31/10

(54) **METHOD FOR PRODUCING C2-3 ALCOHOL**

(30) Priority: 01.11.2022 JP 2022175445; 07.08.2023 JP 2023128832
(71) Applicant: Sumitomo Chemical Company, Limited, Tokyo 103-6020 (JP); Kyoto University, Kyoto-shi, Kyoto 606-8501 (JP)
(72) Inventor: KITAGAWA, Hiroshi, Kyoto-shi, Kyoto 606-8501 (JP); KUSADA, Kohei, Kyoto-shi, Kyoto 606-8501 (JP); KUSHIDA MUKOYOSHI, Megumi, Kyoto-shi, Kyoto 606-8501 (JP); KASHIWABARA, Taigo, Ichihara-shi, Chiba 299-0195 (JP); SATO, Io, Ichihara-shi, Chiba 299-0195 (JP)
(74) Representative: Plasseraud IP
(86) International application number: PCT/JP2023/038568
(87) International publication number: WO 2024/095872

(57) **Abstract**

A method for producing a C2-3 alcohol according to the present disclosure includes: preparing a mixed solution including two or more metal salts of different metal types; obtaining a mixture including a solid containing two or more metals by mixing the mixed solution obtained by the preparing the mixed solution with a reducing agent-containing liquid including a reducing agent; separating the solid from the mixture including the solid obtained by the obtaining the solid; and obtaining the C2-3 alcohol by bringing the solid obtained by the separating the solid into contact with carbon oxide gas and hydrogen gas.

## Description

### CROSS-REFERENCE TO RELATED APPLICATION

This application claims priority to Japanese Patent Application No. 2022-175445 and Japanese Patent Application No. 2023-128832, the disclosures of which are incorporated herein by reference in their entirety.

### FIELD

The present disclosure relates to a method for producing a C2-3 alcohol.

### BACKGROUND

Easy to handle, excellent in durability, and relatively inexpensive, plastics have been in mass production. Most of them are made to be disposable and disposed of as waste, causing problems such as environmental pollution due to microplastics formed of micronized plastics.

In recent years, therefore, methods like reuse, material recycling, chemical recycling, and thermal recycling have been attempted to recycle waste plastics. Material recycling has a problem of degraded quality of plastics through their recycling, the improvement of which is limited. Chemical recycling, in which plastics are chemically decomposed and recycled into petrochemical materials, has in contrast a potential to overcome the limitation of the degraded quality of the plastics caused by the material recycling.

In the process of chemically recycling the waste plastics, an alcohol is produced from carbon oxide gas and hydrogen gas generated by the decomposition of the waste plastics. This alcohol production uses a catalyst. For example, Non-patent Literature 1 discloses that an alcohol is produced from carbon dioxide gas and hydrogen gas under high pressure using a catalyst in which rhodium-iron-lithium is supported on silica gel, which is prepared by the impregnation method. Non-patent Literature 2 discloses that an alcohol is produced from a gas mixture of carbon monoxide and hydrogen under high pressure using a catalyst in which rhodium-iron-cerium is supported on titanium oxide.

### CITATION LIST

### Non-Patent Literature

Non-patent Literature 1: CHEMISTRY AND CHEMICAL INDUSTRY, Vol. 47, No. 10, pp. 1314-1316 (1994)
Non-patent Literature 2: Catalysis Communications, 98, pp. 90-93 (2017)

### SUMMARY

### Technical Problem

In the conventional production methods, alcohols are produced but a selectivity of an alcohol, particularly a selectivity of a C2-3 alcohol, is not sufficient. Therefore, demanded has been a new production method excellent in the selectivity of a C2-3 alcohol.

The present disclosure has been made in view of such circumstances, and an object thereof is to provide a method for producing a C2-3 alcohol relatively excellent in a selectivity of the C2-3 alcohol.

### Solution to Problem

A method for producing a C2-3 alcohol according to the present disclosure includes: preparing a mixed solution including two or more metal salts of different metal types; obtaining a mixture including a solid containing two or more metals by mixing the mixed solution obtained by the preparing the mixed solution with a reducing agent-containing liquid including a reducing agent; separating the solid from the mixture including the solid obtained by the obtaining the solid; and obtaining the C2-3 alcohol by bringing the solid obtained by the separating the solid into contact with carbon oxide gas and hydrogen gas.

### BRIEF DESCRIPTION OF DRAWINGS

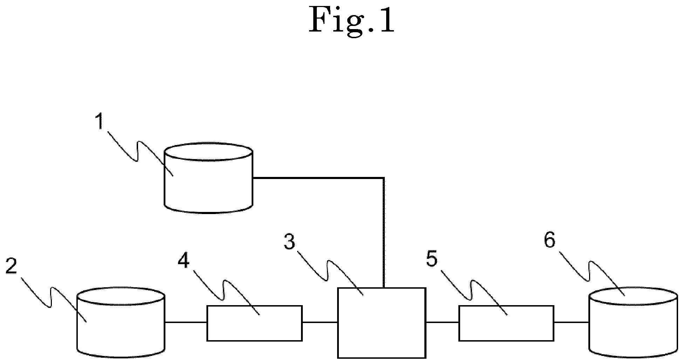

Fig. 1 is a flow chart of steps (1) to (3) in a method for producing a C2-3 alcohol according to this embodiment.

### DESCRIPTION OF EMBODIMENTS

A description will be hereinafter given on an embodiment of the present disclosure, but the present disclosure is not limited to the following embodiments.

### <Method for producing C2-3 alcohol>

A method for producing a C2-3 alcohol according to this embodiment includes steps (1) to (4) below. Fig. 1 is a flow chart of steps (1) to (3) in the method for producing the C2-3 alcohol according to this embodiment.
Step (1): Step of preparing a mixed solution 1 including two or more metal salts of different metal types
Step (2): Step of obtaining a mixture including a solid containing two or more metals by mixing the mixed solution 1 obtained in step (1) with a reducing agent-containing liquid 2 including a reducing agent
Step (3): Step of separating the solid 6 from the mixture including the solid obtained by in step (2)
Step (4): Step of obtaining the C2-3 alcohol by bringing the solid 6 obtained in step (3) into contact with carbon oxide gas and hydrogen gas

As preparing the mixed solution, step (1) is a step of preparing the mixed solution 1 including two or more metal salts of different metal types. The number of metal types in the metal salts is two or more, preferably two to five, more preferably three to five, further preferably three to four.

The mixed solution 1 including the two or more metal salts is preferably the mixed solution 1 including a metal salt of at least one metal selected from the group consisting of 3d metals, 4d metals, and 5d metals. The mixed solution 1 can also include a metal salt of an f metal. The 3d metals refer to Sc, Ti, V, Cr, Mn, Fe, Co, Ni, Cu and Zn. The 4d metals refer to Y, Zr, Nb, Mo, Tc, Ru, Rh, Pd, Ag and Cd. The 5d metals refer to Hf, Ta, W, Re, Os, Ir, Pt, Au and Hg. The f metal refers to any of La, Ce, Pr, Nd, Pm, Sm, Eu, Gd, Tb, Dy, Ho, Er, Tm, Yb and Lu. The mixed solution 1 more preferably includes a metal salt of at least one metal selected from the group consisting of Pd, Ru, Rh, Ag, Os, Ir, Pt, Au, Mo, Ce, Re, W and the 3d metals, further preferably includes a metal salt of at least one metal selected from the group consisting of Pd, Ru, Rh, Ir, Pt, Au, Ag, Mo, Ce, Re, W, Fe, Co, Ni, Cu, Mn, and Zn, particularly preferably includes a metal salt of at least one metal selected from the group consisting of Pd, Ru, Rh, Ir, Pt, Au, Mo, Fe, Co, Ni and Cu.

The metal salt is preferably water-soluble. Examples of the metal salt include a sulfate, a nitrate, an organic acid salt such as an acetate, a carbonate, a halide (a fluoride, a chloride, a bromide, an iodide), a perchlorate, a hydroxide, a complex, and a metal oxoacid (or a salt thereof). The metal salt is preferably a metal halide, a nitrate, an acetate, or a metal oxoacid (or a salt thereof), more preferably a metal halide, an acetate, or a metal oxoacid (or a salt thereof). Any metal salt can be used without particular limitation as long as it is available. The valence of the metal in the metal salt is not particularly limited, and the metal salt is not necessarily a hydrate.

Examples of the metal salt include:
Li salt: e.g., LiOH, LiCl, LiCH₃COO
Mo salt: e.g., MoCl₃, MoCl₅, Mo(CH₃COO)₅
Mn salt: e.g., MnCl₂, MnCl₂·4H₂O, Mn(CH₃COO)₂, Mn(CH₃COO)₂·4H₂O, Mn(NO₃)₂·6H₂O, Mn(SO₄)·5H₂O
Fe salt: e.g., FeCl₂, FeCl₂·4H₂O, FeCl₃, FeCl₃·6H₂O, Fe(CH₃COO)₃, Fe(NO₃)₂·6H₂O, Fe(NO₃)₃·9H₂O
Ru salt: e.g., RuCl₃, RuCl₃·nH₂O, Ru(CH₃COO)₃, Ru(NO₃)₃, Ru(acac)₃ (acac is an acetylacetonato ligand)
Co salt: e.g., CoCl₂, CoCl₂·6H₂O, Co(CH₃COO)₂, Co(NO₃)₂·6H₂O, Co(SO₄)·7H₂O
Rh salt: e.g., RhCl₂, RhCl₃, RhCl₃·3H₂O, Rh₂(CH₃COO)₂, Rh(C₂H₄O₂)ₓ·H₂O (x represents the number 3 or 4), Rh(NO₃)₃·2H₂O, Rh₂(CO₃)₃, Rh₂(SO₄)₃·4H₂O
Ir salt: e.g., IrCl₃·nH₂O, IrCl₄, IrCl₄·nH₂O, Ir(CH₃COO)ₓ (x represents the number 3-4), Ir(acac)₃ (acac is an acetylacetonato ligand), Ir(NO₃)₃
Ni salt: e.g., NiCl₂, NiCl₂·6H₂O, Ni(CH₃COO)₂·4H₂O, Ni(NO₃)₂·6H₂O
Pd salt: e.g., PdCl₂, K₂PdCl₄, Pd(CH₃COO)₂, Pd(NO₃)₂
Pt salt: e.g., PtCl₂, PtCl₄, K₂PtCl₄, K₂PtCl₆, Pt(CH₃COO)₂, Pt(NO₃)₂
Cu salt: e.g., CuCl, CuCl₂, CuCl₂·2H₂O, Cu(CH₃COO)₂, Cu(CH₃COO)₂·H₂O, Cu(NO₃)₂·3H₂O
Ag salt: e.g., Ag(CH₃COO), AgNO₃
Au salt: e.g., AuCl₃, HAuCl₄·3H₂O, Au(CH₃COO)₃, Au(NO₃)₃
Ce salt: e.g., CeCl₃·7H₂O
Na salt: e.g., NaOH, NaCl
La salt: e.g., LaCl₃·7H₂O
Zn salt: e.g., Zn(NO₃)₂·6H₂O

The metal salt is preferably at least one selected from the group consisting of LiCl, MnCl₂·4H₂O, FeCl₂·4H₂O, RuCl₃·nH₂O, CoCl₂·6H₂O, RhCl₃·3H₂O, Rh₂(CH₃COO)₂, IrCl₃·nH₂O, IrCl₄·nH₂O, NiCl₂, NiCl₂·6H₂O, K₂PdCl₄, K₂PtCl₄, K₂PtCl₆, CuCl₂·2H₂O, AgNO₃, CeCl₃·7H₂O, NaCH₃COO, Na₂MoO₄·2H₂O, Na₂WO₄·2H₂O, LaCl₃·7H₂O, and Zn(NO₃)₂·6H₂O.

The mixed solution 1 can further include a solvent for dissolving the two or more metal salts therein. Examples of the solvent include polar solvents such as water, alcohols (e.g., methanol, ethanol, 1-propanol, 2-propanol), polyols (e.g., ethylene glycol, diethylene glycol, triethylene glycol, propylene glycol, glycerin), polyethers (e.g., polyethylene glycol), acetonitrile, acetone, dimethylformamide, dimethyl sulfoxide, and N-methylpyrrolidone. Among these, the solvent is preferably water or an alcohol. These solvents can be individually used, of two or more of them can be used in combination.

The concentration of each metal salt in the mixed solution 1 is preferably 0.001 to 1 mol/L, more preferably 0.025 to 0.1 mol/L.

The mixed solution 1 including the two or more metal salts can be prepared, for example, by dissolving the two or more metal salts collectively in the solvent, or by dissolving the two or more metal salts respectively in separate portions of the solvent and then mixing the separate portions of the solvent in which the two or more metal salts are respectively dissolved. An acid or a base can be added for pH adjustment. As the acid, hydrochloric acid, sulfuric acid, acetic acid, nitric acid, or the like can be used, and hydrochloric acid can be preferably used. As the base, sodium hydroxide, potassium hydroxide, cesium hydroxide, ammonia water, or the like can be used, and sodium hydroxide can be preferably used.

As obtaining the mixture, step (2) is a step of obtaining the mixture including the solid containing the two or more metals by mixing the mixed solution 1 obtained in step (1) with the reducing agent-containing liquid 2 including the reducing agent. More specifically, as shown in Fig. 1, step (2) is a step of obtaining the mixture including the solid containing the two or more metals by feeding the mixed solution 1 and the reducing agent-containing liquid 2 to a mixing part 3 for mixing.

In one aspect of step (2), the mixed solution 1 and the reducing agent-containing liquid 2 are mixed to cause the mixture to react under pressure. In the case where at least one selected from the group consisting of the solvent included in the mixed solution 1, the solvent included in the reducing agent-containing liquid 2, and the reducing agent included in the reducing agent-containing liquid 2 has a boiling point lower than a reaction temperature, the mixture is caused to react under pressure so as to be heated to an appropriate reaction temperature.

In one aspect, it is preferable that the mixed solution 1 and the reducing agent-containing liquid 2 be fed to the mixing part 3 to cause the mixture to react in a continuous flow mode. The mixed solution 1 and the reducing agent-containing liquid 2 can be individually fed to the mixing part 3. The mixed solution 1 and the reducing agent-containing liquid 2 are preferably fed to the mixing part 3 under pressure. The mixed solution 1 and the reducing agent-containing liquid 2 can be continuously or intermittently fed to the mixing part 3. Examples of a device including the mixing part 3 include a microreactor and a flow reactor. In another aspect, the reducing agent-containing liquid 2 can be heated, and then the mixed solution 1 can be mixed therewith by being dropped by a pump or sprayed by a spray device.

A base can be fed to the mixing part 3 for pH adjustment. As the base, sodium hydroxide, potassium hydroxide, cesium hydroxide, ammonia water, or the like can be used, and sodium hydroxide can be preferably used. These are each used in a form of an aqueous solution or an alcohol solution, preferably in a form of an aqueous solution. The base can be mixed with the reducing agent-containing liquid 2 before the reducing agent-containing liquid 2 is fed to the mixing part 3, or can be directly fed to the mixing part 3 either continuously or intermittently. In another aspect, the base can be fed from another line immediately before the mixed solution 1 or the reducing agent-containing liquid 2 flows into the mixing part 3, or the base already mixed with the mixed solution 1 or the reducing agent-containing liquid 2 can flow into the mixing part 3.

The pressure to be applied to each of the mixed solution 1 and the reducing agent-containing liquid 2 for being fed to the mixing part 3 is preferably 0 to 40 MPa-G, more preferably 0 to 35 MPa-G. The pressure within the mixing part 3 is preferably 0 to 40 MPa-G, more preferably 0 to 35 MPa-G. The temperature (reaction temperature) of the solution in which the mixed solution 1 and the reducing agent-containing liquid 2 are mixed within the mixing part 3 is preferably 100 to 500 °C, more preferably 150 to 400 °C, further preferably 200 to 400 °C. The mixing part 3 preferably includes a heater for heating to the reaction temperature. The temperature of the heater can be set to a temperature at which the temperature of the mixing part 3 is a desired temperature.

In one aspect, the mixed solution 1 and the reducing agent-containing liquid 2 can be prepared first and then fed under pressure to the mixing part 3. The feeding to the mixing part 3 can be performed using, for example, a pump.

In step (2), the reducing agent-containing liquid 2 before being fed to the mixing part 3 can be preheated in a heating part 4. The reducing agent-containing liquid 2 is preferably preheated at ambient pressure or under pressure, more preferably preheated to the reaction temperature under pressure. The reducing agent can be heated at ambient pressure when it has a boiling point higher than the reaction temperature, but cannot be heated to the reaction temperature at ambient pressure when it has a boiling point lower than the reaction temperature, in which case the heating is performed under pressure. When the reducing agent is volatile, pressurizing can prevent the reducing agent from evaporating.

In step (2), the temperature of the mixed solution 1 before being fed to the mixing part 3 can be room temperature, but can be preheated to a temperature higher than room temperature by a heating part (not shown).

What is important in the method for producing the C2-3 alcohol according to this embodiment is temperature control of a solution in which the mixed solution 1 and the reducing agent-containing liquid 2 are mixed in the mixing part 3, and it is preferable to adjust a heater temperature of the mixing part 3, and the temperatures of the mixed solution 1 and the reducing agent-containing liquid 2, which are to be fed to the mixing part 3, to keep the temperature of the solution within an appropriate range.

The temperature of the mixed solution 1 to be fed to the mixing part 3 is preferably 5 to 200 °C, more preferably 10 to 150 °C, further preferably 15 to 100 °C, particularly preferably 15 to 50 °C. The temperature of the reducing agent-containing liquid 2 to be fed to the mixing part 3 is preferably 100 to 500 °C, more preferably 150 to 400 °C, further preferably 200 to 400 °C. It is preferable in one aspect that at least one selected from the group consisting of the mixed solution 1 obtained in step (1) and the reducing agent-containing liquid 2 be mixed while being heated to 100°C or higher.

The reducing agent-containing liquid 2 can be used in an amount of 1 or more equivalents relative to the metal salt in terms of the reducing agent component. The reducing agent-containing liquid 2 can preferably be used in an amount of 2 or more equivalents, more preferably 3 or more equivalents, further preferably 5 or more equivalents, in terms of the reducing agent component.

When the solid precipitates in the mixing part 3, a reaction solution including the solid can be discharged from the mixing part 3, the solid therein can be collected by, e.g., filtration or centrifugal separation, and the reaction solution with the solid removed therefrom can be returned to the mixing part 3 for further reaction.

Examples of the reducing agent included in the reducing agent-containing liquid 2 include metal hydrides such as NaBH₄, LiBH₄, LiAlH₄, LiBEt₃H, and DIBAL; alcohols; glycols; glycerins; and nitrogen-containing compounds. Preferable as the metal hydrides are NaBH₄ and LiBH₄. Examples of the alcohols include methanol, ethanol, 1-propanol, 2-propanol, 1-butanol, and 2-butanol. Examples of the glycols include ethylene glycol, diethylene glycol, triethylene glycol, tetraethylene glycol, propylene glycol, dipropylene glycol, tripropylene glycol, ethylene glycol monoethyl ether, diethylene glycol monoethyl ether, and propylene glycol monomethyl ether. Examples of the glycerins include glycerin, diglycerin, triglycerin, and decaglycerin. Examples of the nitrogen-containing compounds include urea and oleylamine.

The reducing agent is preferably an alcohol or a glycol. The boiling point of the reducing agent is preferably 10 to 300 °C, more preferably 40 to 300 °C, further preferably 60 to 300 °C. Among these, preferable as the reducing agent is ethanol, ethylene glycol, or triethylene glycol. The reducing agent can be individually used, or a plurality of the reducing agents can be used in combination.

The reducing agent-containing liquid 2 can be made of the reducing agent, and can further include water. When the reducing agent-containing liquid 2 includes the reducing agent and water, a mixing ratio between the reducing agent and water is, in reducing agent : water, of preferably 1-99% : 99-1%, more preferably 3-90% : 97-10%, further preferably 5-80% : 95-20%, by mass ratio.

In step (2), the mixture including the solid containing the two or more metals obtained by the reaction in the mixing part 3 can be cooled in a cooling part 5. In this case, the cooled mixture is used in step (23), which will be discussed later, or in step (3).

The solid containing the two or more metals can be covered with a surface protectant. Examples of the surface protectant include polymers such as polyvinylpyrrolidone (PVP) and polyethylene glycol (PEG), amines such as oleylamine, and carboxylic acids such as oleic acid. When the surface protectant is used, the surface protectant is included in the solution obtained by mixing the mixed solution 1 and the reducing agent-containing liquid 2, at a concentration of preferably 0.01 to 100 times, more preferably 0.5 to 50 times, further preferably 1 to 10 times by mass ratio, relative to the total amount of the metal salt. The surface protectant can be included in the mixed solution 1, can be included in the reducing agent-containing liquid 2, can be included in both the mixed solution 1 and the reducing agent-containing liquid 2.

As separating the solid, step (3) is a step of separating the solid 6 from the mixture including the solid obtained in step (2).

The method for separating the solid 6 is not limited. Examples of the separation method include decompression drying, centrifugal separation, filtration, sedimentation, reprecipitation, and separation by a powder separator (cyclone).

The content of each metal atom in the solid 6 separated in step (3) is preferably 1 mol% or more where the total amount of all metal atoms in the solid 6 is 100 mol%. For example, when the solid 6 includes two kinds of metal atoms, the content of each of the two kinds of metal atoms in the solid 6 is preferably 1-99 mol%, more preferably 5-95 mol%, further preferably 10-90 mol%. When the solid 6 includes three kinds of metal atoms, the content of each of the three kinds of metal atoms in the solid 6 is preferably 1-98 mol%, more preferably 5-90 mol%, further preferably 10-80 mol%. When the solid 6 includes four kinds of metal atoms, the content of each of the four kinds of metal atoms in the solid 6 is preferably 1-97%, more preferably 5-85 mol%, further preferably 10-70 mol%. When the solid 6 includes five kinds of metal atoms, the content of each of the five kinds of metal atoms in the solid 6 is preferably 1-96 mol%, more preferably 5-80 mol%, further preferably 10-60 mol%. The content of each kind of metal atoms in the solid 6 can be determined by, for example, ICP analysis or XRF analysis.

The solid 6 separated in step (3) can be supported on a catalyst support.

When the solid 6 separated in step (3) is supported on a catalyst support, one aspect includes the following step (23) of bringing the mixture including the solid obtained by obtaining the mixture into contact with the catalyst support, between step (2) and step (3).

### Step (23): Step of bringing the mixture including the solid obtained in step (2) into contact with a catalyst support

In step (23), it is preferable that at least part of the mixture cooled in the cooling part 5 be brought into contact with the catalyst support. The catalyst support for the contact can be molded.

When the solid 6 separated in step (3) is supported on the catalyst support, in another aspect, at least one selected from the group consisting of the mixed solution 1 obtained in step (1) and the reducing agent-containing liquid 2 includes the catalyst support. A dispersed solution in which the catalyst support is dispersed in the at least one selected from the group consisting of the mixed solution 1 and the reducing agent-containing liquid 2 is fed to the mixing part 3. In such a case, the surface protectant can be used, or does not have to be used.

The solid 6 supported on the catalyst support or the solid 6 not supported on the catalyst support can be separated by being collected and dried by a known method such as decompression drying, centrifugal separation, or filtration.

Examples of the catalyst support include alumina, zirconia, titania, ceria, silica, silica-alumina, calcia, magnesia, ceria-zirconia, lantana, lantana-alumina, tin oxide, tungsten oxide, aluminosilicate, aluminophosphate, borosilicate, phosphotungstic acid, hydroxyapatite, hydrotalcite, perovskite, cordierite, mullite, or a composite oxide including one, or two or more of these; silicon carbide; activated carbon; carbon black; acetylene black; a carbon nanotube; and a carbon nanohorn. The catalyst support can include one, or two or more of these.

The catalyst support has an average particle size of preferably 10 nm or more, more preferably 15 nm or more, further preferably 50 nm or more, particularly preferably 100 nm or more. The average particle size of the catalyst support is preferably 1000 µm or less, more preferably 800 µm or less, further preferably 500 µm or less, particularly preferably 100 µm or less. The average particle size of the catalyst support falling within the above range enables the solid 6 to be well dispersibly supported on the catalyst support. The average particle size of the catalyst support can be measured with a general particle size distribution measuring apparatus.

When the solid 6 is to be supported on the catalyst support, the mass of the solid 6 based on 100 mass parts of the catalyst support is preferably 0.01 mass part or more, more preferably 0.05 mass part or more. The mass of the solid 6 based on 100 mass parts of the catalyst support is preferably 20 mass parts or less, more preferably 10 mass parts or less, further preferably 5 mass parts or less, particularly preferably 3 mass parts or less. When the mass of the solid 6 based on 100 mass parts of the catalyst support is equal to or greater than the lower limit above, a sufficient amount of catalytic metal is supported on the catalyst support to maintain a conversion ratio of feedstock gas. When the mass of the solid 6 based on 100 mass parts of the catalyst support is equal to or less than the upper limit above, catalytic metal can be made in a uniform and highly dispersed state without decreasing the selectivity of the C2-3 alcohol and without decreasing the conversion ratio of the feedstock gas. Hereinafter, the term "solid 6" refers to either or both of the form in which the solid 6 is not supported on the catalyst support and the form in which the solid 6 is supported on the catalyst support and includes the catalyst support.

As obtaining the C2-3 alcohol, step (4) is a step of obtaining the C2-3 alcohol by bringing the solid 6 into contact with carbon oxide gas and hydrogen gas. That is, step (4) is a step of obtaining ethanol and/or propanol (1-propanol and 2-propanol).

The carbon oxide gas in step (4) is preferably at least one selected from the group consisting of carbon monoxide and carbon dioxide gas. When both carbon monoxide and carbon dioxide are used as the carbon oxide gas, they can be included at a given ratio. Practically, carbon monoxide gas, carbon dioxide gas, and hydrogen gas as the feedstocks are not actually limited to those derived from waste plastics. Biomass or general waste can be used as gasification feedstock. The feedstocks are not limited to those gasified above. For example, carbon dioxide can be derived from various exhaust gases, combustion gases, incineration gases, or DAC. Hydrogen can be gray hydrogen, blue hydrogen, green hydrogen, or turquoise hydrogen.

In an environment in which step (4) is carried out, any component other than carbon oxide gas and hydrogen gas can be present to the extent not affecting the production of the C2-3 alcohol. Examples of the component include saturated and unsaturated hydrocarbons such as methane, ethane, propane, ethylene, and propylene; nitrogen; and noble gas elements.

In the environment in which step (4) is carried out, water can be present in addition to the feedstocks, i.e., carbon oxide gas and hydrogen gas. Essentially, less water is more preferable in the production of the C2-3 alcohol. However, in the environment in which step (4) is carried out, the presence of a certain amount of water still maintains the stability of the solid 6.

The total content of carbon oxide gas and hydrogen gas in the environment in which step (4) is carried out is preferably 50 vol% or more, more preferably 80 vol% or more, further preferably 90 vol% or more, and can be 100 vol%, in terms of more efficiently obtaining the C2-3 alcohol.

In the environment in which step (4) is carried out, a ratio of hydrogen gas to carbon oxide gas (i.e., hydrogen gas / carbon oxide gas in volume ratio) is preferably 1.0 or more and 4.0 or less, more preferably 1.5 or more and 4.0 or less, in terms achieving a higher space-time yield of the C2-3 alcohol. This ratio can be adjusted by a known method, for example, by additionally mixing at least one gas selected from the group consisting of carbon monoxide, carbon dioxide, and hydrogen to a mixed gas (mixture of carbon oxide gas and hydrogen gas), or by removing part of at least one gas selected from the group consisting of carbon monoxide, carbon dioxide, and hydrogen from the mixed gas. The ratio can be adjusted further using a carbon dioxide separating apparatus as necessary.

Step (4) is preferably carried out in a reactor filled with the solid 6. Examples of reactor include a fixed bed reactor, a fluidized bed reactor, and a moving bed reactor.

The form of the solid 6 used in step (4) can be powder or a molded product. The shape of the molded product is not particularly limited and can be, e.g., a particulate shape, a cylindrical shape, or a ring shape. A method for molding the solid 6 is not particularly limited and can be, e.g., tableting, compression molding, and extrusion molding.

When the reactor is filled with the solid 6, the solid 6 can be mixed with various diluents inert to carbon oxide gas, hydrogen gas, and reaction products. Examples of the diluents include copper, alumina, zirconia, quartz, glass, and silicon carbide. The diluents can be molded into, for example, a granular shape, a spherical shape, or a cylindrical shape, or can have an irregular shape.

The amount of the solid 6 for use is not particularly limited, and can be adjusted appropriately in view of other reaction conditions. A single kind of the solid 6 can be individually used, or two or more kinds of the solid 6 can be used in combination. In the case where two or more kinds are used in combination, the combination and the ratio thereof can be adjusted appropriately according to the purpose.

In step (4), the solid 6 can be subjected to reduction treatment before the solid 6 is brought into contact with carbon oxide gas and hydrogen gas. As the reduction treatment, a method for bringing the solid 6 into contact with a gas containing hydrogen is simple and preferable. In so doing, the treatment temperature is preferably 100 to 600 °C.

In step (4), the solid 6 is brought into contact with carbon oxide gas and hydrogen gas preferably under a heating condition, more preferably at 150 °C or higher, further preferably at 200 °C or higher. The temperature at which these are brought into contact with each other is preferably 150 to 500 °C, more preferably 200 to 400 °C.

The reaction in step (4) can be carried out under an ambient pressure (0 MPa-G) condition, or can be carried out under a pressurized condition. When carried out under the pressurized condition, the pressure is preferably 0.1 to 12 MPa-G, more preferably 0.1 to 10 MPa-G, further preferably 0.1 to 8 MPa-G.

When step (4) is carried out using, for example, the fixed bed reactor, carbon oxide gas and hydrogen gas can be fed through an inlet of the reactor for reaction while the temperature is adjusted, and the reaction product and unreacted feedstock can be collected through an outlet of the reactor. The target product and the unreacted feedstock are generally collected as mixed gas, but the C2-3 alcohol can be separated from the mixed gas by, for example, cooling the gas. The unreacted feedstock gas can be recycled back to the inlet of the reactor after being adjusted for the ratio of hydrogen gas to carbon oxide gas as necessary.

The C2-3 alcohol obtained in step (4) can be converted to an olefin by a known method. The C2-3 alcohol as the feedstock can be used after being purified and separated into necessary components, or can be used in the form as obtained in step (4). An alcohol obtained by a method other than the method for producing the C2-3 alcohol according to this embodiment can be added separately. The alcohol in the alcohol-containing feedstock can include water and/or other oxygen-containing compounds, or can have water and/or other oxygen-containing components intentionally added thereto.

In step (4), the products other than the C2-3 alcohol (e.g., oxygenated products such as acetic acid and acetaldehyde, and esters such as ethyl acetate, methyl acetate, and methyl formate) can also be generated. Thus, the method for producing the C2-3 alcohol according to this embodiment can include step (4') (alcoholization step) of hydrogenating the products other than the C2-3 alcohol for conversion into an alcohol. Examples of step (4') include a method for conversion into an alcohol by bringing an oxygenated product including acetaldehyde, acetic acid, or the like into contact with a hydrogenation catalyst.

Any known catalyst can be used as the hydrogenation catalyst in step (4'). Examples of the known catalyst include copper, copper-zinc, copper-chromium, copper-zinc-chromium, iron, rhodium-iron, rhodium-molybdenum, palladium, palladium-iron, palladium-molybdenum, iridium-iron, rhodium-iridium-iron, iridium-molybdenum, rhenium-zinc, platinum, nickel, cobalt, ruthenium, rhodium oxide, palladium oxide, platinum oxide, and ruthenium oxide. These hydrogenation catalysts can be supported catalysts supported on a catalyst support similar to the aforementioned catalyst support used for the solid 6. The hydrogenation catalyst used in step (4') and the solid 6 can be mixed with each other for use, can be filled respectively in multiple separate stages so as to form layers within the single reactor, or can be filled respectively in separate reactors connected to each other for use.

Step (4) can also include a step of bringing at least part of the alcohol-containing product into contact with a base to remove acidic substances.

The method for producing the C2-3 alcohol according to this embodiment includes steps (1) to (4) to thereby enable a relatively excellent selectivity of the C2-3 alcohol. The method for producing the C2-3 alcohol according to this embodiment can still achieve a relatively excellent selectivity of the C2-3 alcohol even when the reaction in step (4) is carried out under a low pressure condition of less than 1 MPa-G.

The method for producing the C2-3 alcohol according to this embodiment is not limited to the aforementioned embodiment, and various modifications can be made without departing from the gist of the disclosure in the present application.

The present disclosure includes the following aspects:
[1] A method for producing a C2-3 alcohol, the method including steps (1) to (4) below:
   Step (1): Step of preparing a mixed solution including two or more metal salts of different metal types
   Step (2): Step of obtaining a mixture including a solid containing two or more metals by mixing the mixed solution obtained in step (1) with a reducing agent-containing liquid including a reducing agent
   Step (3): Step of separating the solid from the mixture including the solid obtained by in step (2)
   Step (4): Step of obtaining the C2-3 alcohol by bringing the solid obtained in step (3) into contact with carbon oxide gas and hydrogen gas
[2] The method for producing the C2-3 alcohol according to [1] above, the method including step (23) below between step (2) and step (3):
   Step (23): Step of bringing the mixture including the solid obtained in step (2) into contact with a catalyst support
[3] The method for producing the C2-3 alcohol according to [1] above, in which at least one selected from the group consisting of the mixed solution obtained in step (1) and the reducing agent-containing liquid includes a catalyst support.
[4] The method for producing the C2-3 alcohol according to any one of [1] to [3] above, in which, in step (2), at least one selected from the group consisting of the mixed solution obtained in step (1) and the reducing agent-containing liquid is mixed while being heated to 100 °C or higher.
[5] The method for producing the C2-3 alcohol according to any one of [1] to [4] above, in which the reducing agent is an alcohol.
[6] The method for producing the C2-3 alcohol according to any one of [1] to [5] above, in which the mixed solution including the two or more metal salts is a mixed solution including a metal salt of at least one metal selected from the group consisting of 3d metals, 4d metals, and 5d metals.
[7] The method for producing the C2-3 alcohol according to any one of [1] to [6] above, in which, in step (4), the solid obtained in step (3) is brought into contact with the carbon oxide gas and the hydrogen gas at 200 °C or higher.
[8] The method for producing the C2-3 alcohol according to any one of [1] to [7] above, in which the carbon oxide gas is at least one selected from the group consisting of carbon monoxide gas and carbon dioxide gas.

The method for producing the C2-3 alcohol according to the present disclosure is not limited to the aforementioned embodiment, and various modifications can be made without departing from the gist of the present disclosure. The method of producing the C2-3 alcohol according to the present disclosure is not limited by the operations and effects of the aforementioned embodiment, either. That is, the embodiments disclosed herein should be considered to be illustrative in all respects and not restrictive. The scope of the present disclosure is defined by the claims rather than by the above descriptions. The scope of the present disclosure is intended to include meanings equivalent to the claims and all changes without departing from the claims.

### EXAMPLES

The present disclosure will be hereinafter described by way of examples, but the present disclosure is not limited by these examples.

### (Example 1)

### <Preparation of catalyst (solid)>

25 mL of dehydrated ethylene glycol and 0.113 mL of 35 mass % hydrochloric acid were mixed together to prepare a solution (1). 389.6 mg of iron (II) chloride tetrahydrate was mixed with 5.0 mL of the solution (1) to obtain a solution (2). Subsequently, 83.1 mg of lithium chloride was mixed with 5.0 mL of the solution (1) to obtain a solution (3), and 516.1 mg of rhodium chloride trihydrate was mixed with 5.0 mL of the solution (1) to obtain a solution (4). The entire amount of the solution (2) was added to the solution (4), and then the entire amount of the solution (3) was added thereto to obtain a solution (5). 10 mL of the solution (1) was added to 6.175 g of a SiO₂ catalyst support (specific surface area of 250 m²/g), and then the entire amount of the solution (5) was added thereto to obtain a mixture (1). 4.0 g of sodium hydroxide was added for dissolution to 100 mL of ethylene glycol heated to 195 °C, and the mixture (1) was dropped thereon. The heating was stopped one minute after the end of the dropping, and another 100 mL of ethylene glycol was added and cooled to 50 °C to obtain a mixture (2). The resulting mixture (2) was divided into 10 mL batches in respective centrifuge tubes, and 20 mL of acetone was added to each of the centrifuge tubes for centrifugal separation (at 7000 rpm) to remove a supernatant. Next, an operation that 5 mL of ion exchange water and 15 mL of acetone were added for another centrifugal separation (at 7000 rpm) to remove a supernatant was carried out three times in total. Then, 5 mL of ethanol and 15 mL of diethyl ether were added for centrifugal separation (at 7000 rpm) to remove a supernatant and obtain a solid. The solids obtained in the respective centrifuge tubes were mixed together and then dried at room temperature under reduced pressure for 12 hours to obtain a FeLiRh/SiO₂ catalyst (a).

### <Production of ethanol and/or propanol>

1.0 g of the FeLiRh/SiO₂ catalyst (a) was filled in a quartz reaction tube having an inner diameter of 12 mm to form a reaction bed. The reaction bed was heated at a reduction temperature of 300 °C for one hour while flowing 100 NmL/min of nitrogen gas containing 10 volume % of hydrogen gas at ambient pressure through the reaction bed, to subject the catalyst to reduction treatment. The unit NmL/min indicates a flow rate converted based on 1 atm at 0 °C. Next, 50 NmL/min of mixed gas (volume ratio: CO₂ / H₂ = 1 / 3) as the feedstock gas flew at 0.8 MPa-G at a reaction temperature of 300 °C to produce ethanol and/or propanol. Note that G represents gauge pressure. The gas flowing through the reaction bed was analyzed by gas chromatography. From the data obtained, the CO₂ conversion ratio (mol%) and the selectivity (mol%, carbon basis) of the products (methane, CO, EtOH and PrOH) were calculated. These results are shown in Table 1.

### (Examples 2 to 4)

Ethanol and/or propanol were produced using the FeLiRh/SiO₂ catalyst (a) in the same manner as in Example 1, except that the reduction temperature and the reaction temperature were changed as shown in Table 1.

### (Example 5)

### <Preparation of catalyst (solid)>

25 mL of dehydrated ethylene glycol and 0.113 mL of 35 mass % hydrochloric acid were mixed together to prepare a solution (1). 389.6 mg of iron (II) chloride tetrahydrate was mixed with 5.0 mL of the solution (1) to obtain a solution (2). Next, 483.1 mg of cerium chloride was mixed with 5.0 mL of the solution (1) to obtain a solution (3), and 516.1 mg of rhodium chloride trihydrate was mixed with 5.0 mL of the solution (1) to obtain a solution (4). The entire amount of the solution (2) was added to the solution (4), and then the entire amount of the solution (3) was added thereto to obtain a solution (5). 10 mL of the solution (1) was added to 6.175 g of a TiO₂ catalyst support (specific surface area of 92 m²/g), and then the entire amount of the solution (5) was added thereto to obtain a mixture (1). 4.0 g of sodium hydroxide was added for dissolution to 100 mL of ethylene glycol heated to 195 °C, and the mixture (1) was dropped thereon. The heating was stopped one minute after the end of the dropping, and another 100 mL of ethylene glycol was added and cooled to 50 °C to obtain a mixture (2). The resulting mixture (2) was divided into 10 mL batches in respective centrifuge tubes, and 20 mL of acetone was added to each of the centrifuge tubes for centrifugal separation (at 7000 rpm) to remove a supernatant. Next, an operation that 5 mL of ion exchange water and 15 mL of acetone were added for another centrifugal separation (at 7000 rpm) to remove a supernatant was carried out three times in total. Then, 5 mL of ethanol and 15 mL of diethyl ether were added for centrifugal separation (at 7000 rpm) to remove a supernatant and obtain a solid. The solids obtained in the respective centrifuge tubes were mixed together and then dried at room temperature under reduced pressure for 12 hours to obtain a CeFeRh/TiO₂ catalyst (b).

### <Production of ethanol and/or propanol>

1.0 g of the CeFeRh/TiO₂ catalyst (b) was filled in a quartz reaction tube having an inner diameter of 12 mm to form a reaction bed. The reaction bed was heated at a reduction temperature of 300 °C for one hour while flowing 100 NmL/min of nitrogen gas containing 10 volume % of hydrogen gas at ambient pressure through the reaction bed, to subject the catalyst to reduction treatment. Next, 50 NmL/min of mixed gas (volume ratio: CO / H₂ = 1 / 2) as the feedstock gas flew at 0.8 MPa-G at a reaction temperature of 300 °C to produce ethanol and/or propanol. Note that G represents gauge pressure. The gas flowing through the reaction bed was analyzed by gas chromatography. From the data obtained, the CO conversion ratio (mol%) and the selectivity (mol%, carbon basis) of the products (methane, CO₂, EtOH and PrOH) were calculated. These results are shown in Table 1.

### (Examples 6 to 8)

Ethanol and/or propanol were produced using the CeFeRh/TiO₂ catalyst (b) in the same manner as in Example 5, except that the reduction temperature and the reaction temperature were changed as shown in Table 1.

### (Example 9)

### <Preparation of catalyst (solid)>

15 mL of ion exchange water and 60.4 mg of silver nitrate were mixed together to obtain a solution (1). Then, 99.6 mg of rhodium (III) acetate was mixed with 15 mL of ion exchange water to obtain a solution (2). The solution (2) was mixed with the solution (1) to obtain a solution (3). 10 mL of ion exchange water was added to 1.425 g of the SiO₂ catalyst support (specific surface area of 250 m²/g), to which the entire amount of the solution (3) was added and stirred for 15 minutes to obtain a mixture (1). The mixture (1) was sprayed on 300 mL of ethylene glycol heated to 175 °C. The heating was stopped 10 minutes after the end of the spraying, and the mixture was cooled to 50 °C to obtain a mixture (2). The resulting mixture (2) was divided into 10 mL batches in respective centrifuge tubes, and 20 mL of acetone was added to each of the centrifuge tubes for centrifugal separation (at 7000 rpm) to remove a supernatant. Next, an operation that 5 mL of ion exchange water and 15 mL of acetone were added for another centrifugal separation (at 7000 rpm) to remove a supernatant was carried out three times in total. Then, 5 mL of ethanol and 15 mL of diethyl ether were added for centrifugal separation (at 7000 rpm) to remove a supernatant and obtain a solid. The solids obtained in the respective centrifuge tubes were mixed together and then dried at room temperature under reduced pressure for 12 hours to obtain an AgRh/SiO₂ catalyst (c).

### <Production of ethanol and/or propanol>

Ethanol and/or propanol were produced in the same manner as in Example 5, except that the AgRh/SiO₂ catalyst (c) was used and the reduction temperature and the reaction temperature were changed as shown in Table 1.

### (Example 10)

### <Preparation of catalyst (solid)>

25 mL of dehydrated ethylene glycol and 0.113 mL of 35 mass % hydrochloric acid were mixed together to prepare a solution (1). 232.3 mg of sodium molybdate dihydrate was mixed with 5.0 mL of the solution (1) to obtain a solution (2). Then, 228.2 mg of nickel (II) chloride was mixed with 5.0 mL of the solution (1) to obtain a solution (3), and 316.7 mg of sodium tungstate dihydrate was mixed with 5.0 mL of the solution (1) to obtain a solution (4). The entire amount of the solution (3), followed by the entire amount of the solution (4), was added to the solution (2) to obtain a solution (5). 10 mL of the solution (1) was added to 6.175 g of an Al₂O₃ catalyst support (specific surface area of 170 m²/g), to which the entire amount of the solution (5) was further added, to obtain a mixture (1). 4.0 g of sodium hydroxide was added for dissolution to 100 mL of ethylene glycol heated to 195 °C, and the mixture (1) was dropped thereon. The heating was stopped one minute after the end of the dropping, and another 100 mL of ethylene glycol was added and cooled to 50 °C to obtain a mixture (2). The resulting mixture (2) was divided into 10 mL batches in respective centrifuge tubes for centrifugal separation (at 7000 rpm) to remove a supernatant. Next, an operation that 5 mL of ion exchange water was added for another centrifugal separation (at 7000 rpm) to remove a supernatant was carried out three times in total. Then, 5 mL of ethanol was added for centrifugal separation (at 7000 rpm) to remove a supernatant and obtain a solid. The solids obtained in the respective centrifuge tubes were mixed together and then dried at room temperature under reduced pressure for 12 hours to obtain a MoNiW/Al₂O₃ catalyst (d).

### <Production of ethanol and/or propanol>

Ethanol and/or propanol were produced in the same manner as in Example 1, except that the MoNiW/Al₂O₃ catalyst (d) was used and the reduction temperature and the reaction temperature were changed as shown in Table 1.

### (Example 11)

### <Preparation of catalyst (solid)>

The catalyst (solid) was prepared in the same manner as in Example 10, except that 433.6 mg of cobalt (II) chloride hexahydrate was used for the solution (2), 310.7 mg of copper (II) chloride dihydrate for the solution (3), 362.3 mg of iron (II) chloride tetrahydrate for the solution (4), and 6.175 g of the SiO₂ catalyst support (specific surface area of 250 m²/g) in substitution for the Al₂O₃ catalyst support, to obtain a CoCuFe/SiO₂ catalyst (e).

### <Production of ethanol and/or propanol>

Ethanol and/or propanol were produced in the same manner as in Example 1, except that the CoCuFe/SiO₂ catalyst (e) was used and the reduction temperature and the reaction temperature were changed as shown in Table 1.

### (Example 12)

### <Preparation of catalyst (solid)>

The catalyst (solid) was prepared in the same manner as in Example 10, except that 349.7 mg of iron (II) chloride tetrahydrate was used for the solution (2), 299.8 mg of copper (II) chloride dihydrate for the solution (3), and 523.2 mg of zinc nitrate hexahydrate for the solution (4), to obtain a CuFeZn/Al₂O₃ catalyst (f).

### <Production of ethanol and/or propanol>

Ethanol and/or propanol were produced in the same manner as in Example 1, except that the CuFeZn/Al₂O₃ catalyst (f) was used and the reduction temperature and the reaction temperature were changed as shown in Table 1.

### (Example 13)

### <Preparation of catalyst (solid)>

The catalyst (solid) was prepared in the same manner as in Example 10, except that 285.6 mg of copper (II) chloride dihydrate was used for the solution (2), 622.2 mg of lanthanum chloride heptahydrate for the solution (3), 6.175 g of a ZrO₂ catalyst support (specific surface area of 100 m²/g) in substitution for the Al₂O₃ catalyst support, and 5.6 g of potassium hydroxide in substitution for sodium hydroxide, to obtain a CuKLa/ZrO₂ catalyst (g).

### <Production of ethanol and/or propanol>

Ethanol and/or propanol were produced in the same manner as in Example 1, except that the CuKLa/ZrO₂ catalyst (g) was used and the reduction temperature and the reaction temperature were changed as shown in Table 1.

### (Example 14)

### <Preparation of catalyst (solid)>

The catalyst (solid) was prepared in the same manner as in Example 10, except that 429.7 mg of copper (II) chloride dihydrate was used for the solution (2) and 749.8 mg of zinc nitrate hexahydrate for the solution (3), to obtain a CuZn/Al₂O₃ catalyst (h).

### <Production of ethanol and/or propanol>

Ethanol and/or propanol were produced in the same manner as in Example 1, except that the CuZn/Al₂O₃ catalyst (h) was used and the reduction temperature and the reaction temperature were changed as shown in Table 1.

### (Example 15)

### <Preparation of catalyst (solid)>

The catalyst (solid) was prepared in the same manner as in Example 10, except that 631.4 mg of cobalt (II) chloride hexahydrate was used for the solution (2) and 452.3 mg of copper (II) chloride dihydrate for the solution (3), to obtain a CoCu/Al₂O₃ catalyst (i).

### <Production of ethanol and/or propanol>

Ethanol and/or propanol were produced in the same manner as in Example 1, except that the CoCu/Al₂O₃ catalyst (i) was used and the reduction temperature and the reaction temperature were changed as shown in Table 1.

### (Example 16)

### <Preparation of catalyst (solid)>

The catalyst (solid) was prepared in the same manner as in Example 10, except that 483.3 mg of cobalt (II) chloride hexahydrate was used for the solution (2), 530.6 mg of ruthenium chloride n-hydrate for the solution (3), and 6.175 g of the SiO₂ catalyst support (specific surface area of 250 m²/g) in substitution for the Al₂O₃ catalyst support, to obtain a CoRu/ SiO₂ catalyst (j).

### <Production of ethanol and/or propanol>

Ethanol and/or propanol were produced in the same manner as in Example 1, except that the CoRu/SiO₂ catalyst (j) was used and the reduction temperature and the reaction temperature were changed as shown in Table 1.

### (Example 17)

### <Preparation of catalyst (solid)>

The catalyst (solid) was prepared in the same manner as in Example 10, except that 418.3 mg of cobalt (II) chloride hexahydrate was used for the solution (2), 417.0 mg of rhodium chloride trihydrate for the solution (3), and 6.175 g of the SiO₂ catalyst support (specific surface area of 250 m²/g) in substitution for the Al₂O₃ catalyst support, to obtain a CoNaRh/SiO₂ catalyst (k).

### <Production of ethanol and/or propanol>

Ethanol and/or propanol were produced in the same manner as in Example 1, except that the CoNaRh/SiO₂ catalyst (k) was used and the reduction temperature and the reaction temperature were changed as shown in Table 1.

### (Example 18)

### <Preparation of catalyst (solid)>

150 mg of copper (II) chloride dihydrate and 325 mg of iridium chloride n-hydrate were each mixed with 15 mL of water to obtain a solution (1) and a solution (2), respectively. 4.3 g of the Al₂O₃ catalyst support (specific surface area of 170 m²/g) was mixed with 10 mL of water, to which the solution (1) and the solution (2) were added to obtain a mixture (1). 240 mg of sodium hydroxide was dissolved in 2 mL of water, the entire amount of which was added to 300 mL of triethylene glycol to obtain a mixture (2). The mixture (1) was sprayed on the mixture (2) heated to 224 °C. The heating was stopped 10 minutes after the end of the spraying, and the mixture was allowed to cool to room temperature to obtain a mixture (3). The resulting mixture (3) was divided into 10 mL batches in respective centrifuge tubes, and 20 mL of acetone was added to each of the centrifuge tubes for centrifugal separation (at 7000 rpm) to remove a supernatant. Next, an operation that 5 mL of ion exchange water and 10 mL of acetone were added for another centrifugal separation (at 7000 rpm) to remove a supernatant was carried out three times in total. Then, 3 mL of ethanol was added for centrifugal separation (at 7000 rpm) to remove a supernatant and obtain a solid. The solids obtained in the respective centrifuge tubes were mixed together and then dried at room temperature under reduced pressure for 12 hours to obtain a CuIr/Al₂O₃ catalyst (l).

### <Production of ethanol and/or propanol>

Ethanol and/or propanol were produced in the same manner as in Example 5, except that the CuIr/Al₂O₃ catalyst (l) was used and the reduction temperature and the reaction temperature were changed as shown in Table 1.

### (Example 19)

### <Preparation of catalyst (solid)>

The catalyst (solid) was prepared in the same manner as in Example 10, except that 326.0 mg of copper (II) chloride dihydrate was used for the solution (2) and 624.2 mg of potassium tetrachloropalladate for the solution (3), to obtain a CuPd/Al₂O₃ catalyst (m).

### <Production of ethanol and/or propanol>

Ethanol and/or propanol were produced in the same manner as in Example 5, except that the CuPd/Al₂O₃ catalyst (m) was used and the reduction temperature and the reaction temperature were changed as shown in Table 1.

### (Example 20)

### <Preparation of catalyst (solid)>

234 mg of potassium tetrachloropalladate and 189 mg of rhodium chloride trihydrate were each mixed with 15 mL of water to obtain a solution (1) and a solution (2), respectively. 2.8 g of the Al₂O₃ catalyst support (specific surface area of 170 m²/g) was mixed with 10 mL of water, to which the solution (1) and the solution (2) were added to obtain a mixture (1). The mixture (1) was sprayed on 300 mL of triethylene glycol heated to 224 °C. The heating was stopped 10 minutes after the end of the spraying, and the mixture was allowed to cool to room temperature to obtain a mixture (2). The resulting mixture (2) was divided into 10 mL batches in respective centrifuge tubes, and 20 mL of acetone was added to each of the centrifuge tubes for centrifugal separation (at 7000 rpm) to remove a supernatant. Next, an operation that 5 mL of ion exchange water and 10 mL of acetone were added for another centrifugal separation (at 7000 rpm) to remove a supernatant was carried out three times in total. Then, 3 mL of ethanol was added for centrifugal separation (at 7000 rpm) to remove a supernatant and obtain a solid. The solids obtained in the respective centrifuge tubes were mixed together and then dried at room temperature under reduced pressure for 12 hours to obtain a PdRh/Al₂O₃ catalyst (n).

### <Production of ethanol and/or propanol>

Ethanol and/or propanol were produced in the same manner as in Example 5, except that the PdRh/Al₂O₃ catalyst (n) was used and the reduction temperature and the reaction temperature were changed as shown in Table 1.

### (Example 21)

### <Preparation of catalyst (solid)>

The catalyst (solid) was prepared in the same manner as in Example 18, except that 148 mg of copper (II) chloride dihydrate and 361 mg of potassium tetrachloroplatinate were used, to obtain a CuPt/Al₂O₃ catalyst (o).

### <Production of ethanol and/or propanol>

Ethanol and/or propanol were produced in the same manner as in Example 5, except that the CuPt/Al₂O₃ catalyst (o) was used and the reduction temperature and the reaction temperature were changed as shown in Table 1.

### (Example 22)

### <Production of ethanol and/or propanol>

Ethanol and/or propanol were produced in the same manner as in Example 5, except that the CuKLa/ZrO₂ catalyst (g) obtained in Example 13 was used and the reduction temperature and the reaction temperature were changed as shown in Table 1.

### (Example 23)

### <Production of ethanol and/or propanol>

Ethanol and/or propanol were produced in the same manner as in Example 5, except that the CuZn/Al₂O₃ catalyst (h) obtained in Example 14 was used and the reduction temperature and the reaction temperature were changed as shown in Table 1.

### (Example 24)

### <Preparation of catalyst (solid)>

The catalyst (solid) was prepared in the same manner as in Example 10, except that 657.4 mg of cobalt (II) chloride hexahydrate was used for the solution (2) and 656.7 mg of nickel (II) chloride hexahydrate for the solution (3), to obtain a CoNi/Al₂O₃ catalyst (p).

### <Production of ethanol and/or propanol>

Ethanol and/or propanol were produced in the same manner as in Example 5, except that the CoNi/Al₂O₃ catalyst (p) was used and the reduction temperature and the reaction temperature were changed as shown in Table 1.

### (Example 25)

### <Preparation of catalyst (solid)>

The catalyst was prepared in the same manner as in Example 18, except that 230 mg of copper (II) chloride dihydrate and 356 mg of rhodium chloride trihydrate were used, to obtain a CuRh/Al₂O₃ catalyst (q).

### <Production of ethanol and/or propanol>

Ethanol and/or propanol were produced in the same manner as in Example 5, except that the CuRh/Al₂O₃ catalyst (q) was used and the reduction temperature and the reaction temperature were changed as shown in Table 1.

### (Example 26)

### <Production of ethanol and/or propanol>

Ethanol and/or propanol were produced in the same manner as in Example 5, except that the FeLiRh/SiO₂ catalyst (a) obtained in Example 1 was used and the reduction temperature and the reaction temperature were changed as shown in Table 1.

### (Example 27)

### <Production of ethanol and/or propanol>

Ethanol and/or propanol were produced in the same manner as in Example 5, except that the CuFeZn/Al₂O₃ catalyst (f) obtained in Example 12 was used and the reduction temperature and the reaction temperature were changed as shown in Table 1.

### (Example 28)

### <Preparation of catalyst (solid)>

The catalyst was prepared in the same manner as in Example 20, except that 313 mg of potassium tetrachloroplatinate and 199 mg of rhodium chloride trihydrate were used, to obtain a PtRh/Al₂O₃ catalyst (r).

### <Production of ethanol and/or propanol>

Ethanol and/or propanol were produced in the same manner as in Example 5, except that the PtRh/Al₂O₃ catalyst (r) was used and the reduction temperature and the reaction temperature were changed as shown in Table 1.

### (Example 29)

### <Production of ethanol and/or propanol>

Ethanol and/or propanol were produced in the same manner as in Example 5, except that the CoRu/SiO₂ catalyst (j) obtained in Example 16 was used and the reduction temperature and the reaction temperature were changed as shown in Table 1.

### (Example 30)

### <Preparation of catalyst (solid)>

The catalyst (solid) was prepared in the same manner as in Example 10, except that 398.6 mg of cobalt (II) chloride hexahydrate was used for the solution (2), 405.4 mg of sodium molybdate dihydrate for the solution (3), 6.175 g of a CeO₂ catalyst support (specific surface area of 150 m²/g) in substitution for the Al₂O₃ catalyst support, and 5.6 g of potassium hydroxide in substitution for sodium hydroxide, to obtain a CoKMo/CeO₂ catalyst (s).

### <Production of ethanol and/or propanol>

Ethanol and/or propanol were produced in the same manner as in Example 5, except that the CoKMo/CeO₂ catalyst (s) was used and the reduction temperature and the reaction temperature were changed as shown in Table 1.

### (Example 31)

### <Preparation of catalyst (solid)>

The catalyst was prepared in the same manner as in Example 20, except that 188 mg of iridium chloride n-hydrate and 134 mg of rhodium chloride trihydrate were used, to obtain an IrRh/Al₂O₃ catalyst (t).

### <Production of ethanol and/or propanol>

Ethanol and/or propanol were produced in the same manner as in Example 5, except that the IrRh/Al₂O₃ catalyst (t) was used and the reduction temperature and the reaction temperature were changed as shown in Table 1.

### (Example 32)

### <Preparation of catalyst (solid)>

A 0.1 mol/L hydrochloric acid aqueous solution (1) was prepared. 973.8 mg of cobalt (II) chloride hexahydrate was mixed with 15.0 mL of the solution (1) to obtain a solution (2), 615.3 mg of sodium acetate was mixed with 15.0 mL of the solution (1) to obtain a solution (3), and 395.0 mg of rhodium chloride trihydrate was mixed with 15.0 mL of the solution (1) to obtain a solution (4). 52 mL of the solution (1), 2.14 mL of the solution (2), 2.14 mL of the solution (3), and 10.71 mL of the solution (4) were mixed to obtain a mixed solution (5). Using a plunger pump, a 25 vol% ethanol solution (solution (6)) was pumped into a flow reactor at 100 mL/min while being heated to 350 °C by a heater in the middle of a flow path. A 25 mol/L aqueous sodium hydroxide solution was pumped at 10 mL/min using a separate plunger pump to obtain a flow (1) mixed with the heated solution (6). The mixed solution (5) pumped at 20 mL/min by a syringe pump was mixed with the flow (1) to obtain a flow (2), the flow (2) was quenched by quickly passing through the cooling part, and the mixture (1) was discharged from the flow reactor through a back pressure valve. The back pressure valve was controlled to keep a reaction part at 30 MPa-G. After the mixture (1) was brought into contact with a TiO₂ catalyst support (specific surface area of 92 m²/g), the resulting mixture (2) was filtered by suction to obtain a solid. The solid was washed with 50 mL of ion exchange water and dried at room temperature under reduced pressure for 12 hours to obtain a CoNaRh/TiO₂ catalyst (u).

### <Production of ethanol and/or propanol>

Ethanol and/or propanol were produced in the same manner as in Example 5, except that the CoNaRh/TiO₂ (u) was used and the reduction temperature and the reaction temperature were changed as shown in Table 1.

### (Example 33)

### <Production of ethanol and/or propanol>

Ethanol and/or propanol were produced in the same manner as in Example 5, except that the CoCu/Al₂O₃ catalyst (i) obtained in Example 15 was used and the reduction temperature and the reaction temperature were changed as shown in Table 1.

### (Example 34)

### <Preparation of catalyst (solid)>

The catalyst (solid) was prepared in the same manner as in Example 10, except that 398.6 mg of cobalt (II) chloride hexahydrate was used for the solution (2), 405.4 mg of sodium molybdate dihydrate for the solution (3), and 5.6 g of potassium hydroxide in substitution for sodium hydroxide, to obtain a CoKMo/Al₂O₃ catalyst (v).

### <Production of ethanol and/or propanol>

Ethanol and/or propanol were produced in the same manner as in Example 5, except that CoKMo/Al₂O₃ catalyst (v) was used and the reduction temperature and the reaction temperature were changed as shown in Table 1.

### (Example 35)

### <Preparation of catalyst (solid)>

The catalyst (solid) was prepared in the same manner as in Example 10, except that 499.3 mg of cobalt (II) chloride hexahydrate was used for the solution (2), 507.7 mg of sodium molybdate dihydrate for the solution (3), and 6.175 g of the CeO₂ catalyst support (specific surface area of 150 m²/g) in substation for the Al₂O₃ catalyst support, to obtain a CoMo/CeO₂ catalyst (w).

### <Production of ethanol and/or propanol>

Ethanol and/or propanol were produced in the same manner as in Example 5, except that CoMo/CeO₂ catalyst (w) was used and the reduction temperature and the reaction temperature were changed as shown in Table 1.

### (Example 36)

### <Preparation of catalyst (solid)>

The catalyst (solid) was prepared in the same manner as in Example 10, except that 83.6 mg of lithium chloride was used for the solution (2), 390.3 mg of manganese (II) chloride tetrahydrate for the solution (3), 519.3 mg of rhodium chloride trihydrate for the solution (4), and 6.175 g of the SiO₂ catalyst support (specific surface area of 250 m²/g) in substitution for the Al₂O₃ catalyst support, to obtain a LiMnRh/SiO₂ catalyst (aa).

### <Production of ethanol and/or propanol>

Ethanol and/or propanol were produced in the same manner as in Example 5, except that the LiMnRh/SiO₂ catalyst (aa) was used and the reduction temperature and the reaction temperature were changed as shown in Table 1.

### (Example 37)

### <Production of ethanol and/or propanol>

Ethanol and/or propanol were produced in the same manner as in Example 5, except that the CoCuFe/SiO₂ catalyst (e) obtained in Example 11 was used and the reduction temperature and the reaction temperature were changed as shown in Table 1.

### (Example 38)

### <Production of ethanol and/or propanol>

Ethanol and/or propanol were produced in the same manner as in Example 5, except that the CoNaRh/SiO₂ catalyst (k) obtained in Example 17 was used and the reduction temperature and the reaction temperature were changed as shown in Table 1.

### (Example 39)

### <Preparation of catalyst (solid)>

The catalyst was prepared in the same manner as in Example 20, except that 351 mg of potassium tetrachloropalladate, 283 mg of rhodium chloride trihydrate, and the SiO₂ catalyst support (specific surface area of 250 m²/g) were used, to obtain a PdRh/SiO₂ catalyst (ab).

### <Production of ethanol and/or propanol>

Ethanol and/or propanol were produced in the same manner as in Example 5, except that the PdRh/SiO₂ catalyst (ab) was used and the reduction temperature and the reaction temperature were changed as shown in Table 1.

### (Example 40)

### <Preparation of catalyst (solid)>

The catalyst (solid) was prepared in the same manner as in Example 18, except that 230 mg of copper (II) chloride dihydrate and 356 mg of ruthenium chloride n-hydrate were used, to obtain a CuRu/Al₂O₃ catalyst (ac).

### <Production of ethanol and/or propanol>

Ethanol and/or propanol were produced in the same manner as in Example 5, except that the CuRu/Al₂O₃ catalyst (ac) was used and the reduction temperature and the reaction temperature were changed as shown in Table 1.

### (Example 41)

### <Preparation of catalyst (solid)>

The catalyst was prepared in the same manner as in Example 20, except that 290 mg of rhodium chloride trihydrate, 288 mg of ruthenium chloride n-hydrate, and the SiO₂ catalyst support (specific surface area of 250 m²/g) were used, to obtain a RhRu/SiO₂ catalyst (ad).

### <Production of ethanol and/or propanol>

Ethanol and/or propanol were produced in the same manner as in Example 5, except that the RhRu/SiO₂ catalyst (ad) was used and the reduction temperature and the reaction temperature were changed as shown in Table 1.

### (Comparative Example 1)

### <Preparation of catalyst>

A catalyst was prepared according to the method described in Non-patent Literature 1 (CHEMISTRY AND CHEMICAL INDUSTRY, Vol. 47, No. 10, pp. 1314-1316 (1994)). Specifically, 0.3430 g of rhodium nitrate n-hydrate, 0.4882 g of iron nitrate nonahydrate, and 0.0570 g of lithium chloride were mixed with 5.3 mL of water to obtain an aqueous solution. The aqueous solution was dropped onto 3.78 g of the SiO₂ catalyst support (specific surface area of 250 m²/g) for impregnation. The resulting product was placed onto an alumina dish and dried at 110 °C under air for 19 hours, followed by calcination at 500 °C under air for 4 hours to obtain a FeLiRh/SiO₂ catalyst (x).

### <Production of ethanol and/or propanol>

Ethanol and/or propanol were produced in the same manner as in Example 1, except that the FeLiRh/SiO₂ catalyst (x) was used and the reduction temperature and the reaction temperature were changed as shown in Table 1.

### (Comparative Example 2)

### <Preparation of catalyst>

A catalyst was prepared according to the method described in Non-patent Literature 2 (Catalysis Communications, 98, pp. 90-93 (2017)). Specifically, 0.1600 g of rhodium nitrate n-hydrate, 0.2727 g of iron nitrate nonahydrate, and 0.3023 g of cerium nitrate hexahydrate were mixed with 1.4 mL of water to obtain an aqueous solution. The aqueous solution was dropped onto 3.78 g of the TiO₂ catalyst support (specific surface area of 92 m²/g) for impregnation. The resulting product was placed onto an alumina dish and dried at 110 °C under air for 19 hours, followed by calcination at 500 °C under air for 4 hours to obtain a CeFeRh/TiO₂ catalyst (y).

### <Production of ethanol and/or propanol>

Ethanol and/or propanol were produced in the same manner as in Example 5, except that the CeFeRh/TiO₂ catalyst (y) was used and the reduction temperature and the reaction temperature were changed as shown in Table 1.

**Table 1**

| | Catalyst | Feedsto ck gas | Reduction temp.(°C) | Reaction temp. (°C) | Conversion ratio (%) | | Selectivity (%) | | | | |
|---|---|---|---|---|---|---|---|---|---|---|---|
| | | | | | CO₂ | CO | CO₂ | CO | CH₄ | EtOH | PrOH |
| Ex. 1 | FeLiRh/SiO₂(a) | CO₂ | 300 | 300 | 13.08 | - | - | 81.74 | 14.26 | 1.69 | 0.19 |
| Ex. 2 | FeLiRh/SiO₂(a) | CO₂ | 350 | 350 | 24.55 | - | - | 72.22 | 25.53 | 0.45 | 0.06 |
| Ex. 3 | FeLiRh/SiO₂(a) | CO₂ | 300 | 350 | 22.75 | - | - | 71.18 | 25.02 | 1.28 | 0.00 |
| Ex. 4 | FeLiRh/SiO₂(a) | CO₂ | 350 | 300 | 14.89 | - | - | 85.62 | 11.75 | 1.18 | 0.15 |
| Ex. 5 | CeFeRh/TiO₂(b) | CO | 300 | 300 | - | 3.31 | 29.43 | - | 33.24 | 20.20 | 2.69 |
| Ex. 6 | CeFeRh/TiO₂(b) | CO | 300 | 350 | - | 17.62 | 45.57 | - | 39.22 | 3.38 | 0.43 |
| Ex. 7 | CeFeRh/TiO₂(b) | CO | 350 | 300 | - | 5.02 | 39.83 | - | 32.51 | 13.72 | 1.38 |
| Ex. 8 | CeFeRh/TiO₂(b) | CO | 350 | 350 | - | 29.87 | 44.65 | - | 40.81 | 2.28 | 0.20 |
| Ex. 9 | AgRh/SiO₂(c) | CO | 300 | 340 | - | 3.50 | 1.90 | - | 53.10 | 2.00 | 0.00 |
| Ex. 10 | MoNiW/Al₂O₃(d) | CO₂ | 300 | 300 | 1.41 | - | - | 76.9911 | 17.07 | 2.39 | 0.99 |
| Ex. 11 | CoCuFe/SiO₂(e) | CO₂ | 300 | 300 | 8.76 | - | - | 72.5124 | 19.55 | 1.98 | 0.48 |
| Ex. 12 | CuFeZn/Al₂O₃(f) | CO₂ | 300 | 300 | 8.39 | - | - | 95.2647 | 0.86 | 1.80 | 0.41 |
| Ex. 13 | CuKLa/ZrO₂(g) | CO₂ | 300 | 300 | 3.59 | - | - | 90.5411 | 0.00 | 0.91 | 0.65 |
| Ex. 14 | CuZn/Al₂O₃(h) | CO₂ | 300 | 300 | 7.88 | - | - | 95.9434 | 1.55 | 0.56 | 0.35 |
| Ex. 15 | CoCu/Al₂O₃(i) | CO₂ | 300 | 300 | 19.3669 | - | - | 25.2238 | 62.50 | 0.55 | 0.16 |
| Ex. 16 | CoRu/SiO₂(j) | CO₂ | 300 | 300 | 15.1873 | - | - | 60.1945 | 31.15 | 0.50 | 0.14 |
| Ex. 17 | CoNaRh/SiO₂(k) | CO₂ | 300 | 300 | 18.2608 | - | - | 26.4326 | 52.76 | 0.38 | 0.12 |
| Ex. 18 | CuIr/Al₂O₃(l) | CO | 300 | 350 | - | 1.01 | 20.25 | - | 56.72 | 8.63 | 1.30 |
| Ex. 19 | CuPd/Al₂O₃(m) | CO | 300 | 350 | - | 1.53 | 15.54 | - | 29.55 | 8.33 | 0.76 |
| Ex. 20 | PdRh/Al₂O₃(n) | CO | 300 | 350 | - | 1.86 | 8.59 | - | 63.39 | 6.56 | 0.85 |
| Ex. 21 | CuPt/Al₂O₃(o) | CO | 300 | 350 | - | 1.09 | 26.25 | - | 44.87 | 3.25 | 0.91 |
| Ex. 22 | CuKLa/ZrO₂(g) | CO | 300 | 350 | - | 2.96 | 38.69 | - | 20.90 | 2.84 | 2.02 |
| Ex. 23 | CuZn/Al₂O₃(h) | CO | 300 | 350 | - | 1.63 | 44.90 | - | 19.30 | 2.80 | 1.47 |
| Ex. 24 | CoNi/Al₂O₃(p) | CO | 300 | 350 | - | 2.73 | 30.16 | - | 35.51 | 2.18 | 0.73 |
| Ex. 25 | CuRh/Al₂O₃(q) | CO | 300 | 350 | - | 1.92 | 65.34 | - | 12.59 | 2.05 | 1.56 |
| Ex. 26 | FeLiRh/SiO₂(a) | CO | 300 | 350 | - | 27.13 | 43.98 | - | 34.92 | 1.99 | 0.88 |
| Ex. 27 | CuFeZn/Al₂O₃(f) | CO | 300 | 350 | - | 11.68 | 51.10 | - | 19.57 | 1.88 | 0.54 |
| Ex. 28 | PtRh/Al₂O₃(f) | CO | 300 | 350 | - | 3.38 | 5.93 | - | 79.18 | 1.63 | 0.25 |
| Ex. 29 | CoRu/SiO₂(j) | CO | 300 | 350 | - | 14.47 | 26.78 | - | 27.96 | 1.35 | 0.53 |
| Ex. 30 | CoKMo/CeO₂(s) | CO | 300 | 350 | - | 20.54 | 24.52 | - | 28.88 | 1.26 | 0.60 |
| Ex. 31 | IrRh/Al₂O₃(t) | CO | 300 | 350 | - | 3.17 | 6.29 | - | 75.05 | 1.14 | 0.23 |
| Ex. 32 | CoNaRh/TiO₂(u) | CO | 300 | 350 | - | 8.26 | 51.28 | - | 14.80 | 1.05 | 0.86 |
| Ex. 33 | CoCu/Al₂O₃(i) | CO | 300 | 350 | - | 15.27 | 28.05 | - | 40.21 | 0.95 | 0.46 |
| Ex. 34 | CoKMo/Al₂O₃(v) | CO | 300 | 350 | - | 23.63 | 35.06 | - | 35.70 | 0.81 | 0.31 |
| Ex. 35 | CoMo/CeO₂(w) | CO | 300 | 350 | - | 19.12 | 20.77 | - | 31.44 | 0.67 | 0.28 |
| Ex. 36 | LiMnRh/SiO₂(aa) | CO | 300 | 350 | - | 20.21 | 30.38 | - | 52.91 | 0.66 | 0.21 |
| Ex. 37 | CoCuFe/SiO₂(e) | CO | 300 | 350 | - | 33.53 | 33.64 | - | 35.95 | 0.64 | 0.40 |
| Ex. 38 | CoNaRh/SiO₂(k) | CO | 300 | 350 | - | 33.43 | 39.03 | - | 28.35 | 0.45 | 0.44 |
| Ex. 39 | PdRh/SiO₂(ab) | CO | 300 | 350 | - | 10.41 | 6.50 | - | 88.72 | 0.34 | 0.05 |
| Ex. 40 | CuRu/Al₂O₃(ac) | CO | 300 | 350 | - | 33.12 | 6.30 | - | 88.20 | 0.09 | 0.02 |
| Ex. 41 | RhRu/SiO₂(ad) | CO | 300 | 350 | - | 66.26 | 2.99 | - | 95.42 | 0.05 | 0.00 |
| C. Ex. 1 | FeLiRh/SiO₂(x) | CO₂ | 300 | 300 | 15.80 | - | - | 50.25 | 46.99 | 0.28 | 0.00 |
| C. Ex. 2 | CeFeRh/TiO₂(y) | CO | 300 | 350 | - | 53.21 | 46.74 | - | 33.75 | 0.04 | 0.00 |

The results in Table 1 show that Examples 1 to 41 each are more excellent in the selectivity of the C2-3 alcohol than that of Comparative Examples 1 and 2.

### REFERENCE SIGNS LIST

1: Mixed solution
2: Reducing agent-containing liquid
3: Mixing part
4: Heating part
5: Cooling part
6: Solid

## Claims

1. A method for producing a C2-3 alcohol, the method comprising:
preparing a mixed solution comprising two or more metal salts of different metal types;
obtaining a mixture comprising a solid containing two or more metals by mixing the mixed solution obtained by the preparing the mixed solution with a reducing agent-containing liquid including a reducing agent;
separating the solid from the mixture comprising the solid obtained by the obtaining the solid; and
obtaining the C2-3 alcohol by bringing the solid obtained by the separating the solid into contact with carbon oxide gas and hydrogen gas.

2. The method for producing the C2-3 alcohol according to claim 1, the method further comprising bringing the mixture comprising the solid obtained by the obtaining the mixture into contact with a catalyst support, between the obtaining the mixture and the separating the solid.

3. The method for producing the C2-3 alcohol according to claim 1, wherein, in the obtaining the mixture, at least one selected from the group consisting of the mixed solution obtained by the preparing the mixed solution and the reducing agent-containing liquid comprises a catalyst support.

4. The method for producing the C2-3 alcohol according to any one of claims 1 to 3, wherein, in the obtaining the mixture, at least one selected from the group consisting of the mixed solution obtained by the preparing the mixed solution and the reducing agent-containing liquid is mixed while being heated to 100 °C or higher.

5. The method for producing the C2-3 alcohol according to any one of claims 1 to 3, wherein the reducing agent is an alcohol.

6. The method for producing the C2-3 alcohol according to any one claims 1 to 3, wherein the mixed solution comprising the two or more metal salts is a mixed solution comprising a metal salt of at least one metal selected from the group consisting of 3d metals, 4d metals, and 5d metals.

7. The method for producing the C2-3 alcohol according to any one of claims 1 to 3, wherein, in the obtaining the C2-3 alcohol, the solid obtained by the separating the solid is brought into contact with the carbon oxide gas and the hydrogen gas at 200 °C or higher.

8. The method for producing the C2-3 alcohol according to any one of claims 1 to 3, wherein the carbon oxide gas is at least one selected from the group consisting of carbon monoxide gas and carbon dioxide gas.
